# EUROPÄISCHE PATENTSCHRIFT

(11) **EP 2 027 834 B1**
(45) Veröffentlichungstag und Bekanntmachung des Hinweises auf die Patenterteilung: **17.03.2010**
(21) Anmeldenummer: 07021510.8
(22) Anmeldetag: 06.11.2007
(51) Int. Cl.: A61F 5/11

(54) **Vorrichtung zur Nagelkorrektur**
Device for correcting nails
Dispositif destiné à la correction d'ongle

(30) Priorität: 26.07.2007 DE 102007035031
(43) Veröffentlichungstag der Anmeldung: 25.02.2009
(73) Patentinhaber: 3TO GmbH, 82041 Deisenhofen (DE)
(72) Erfinder: Sutor, Norbert, 82041 Deisenhofen (DE); Sutor, Franziska, 80333 München (DE); Sutor, Johannes, 80339 München (DE)
(74) Vertreter: Müller, Bernhard

(56) Entgegenhaltungen:
- DE-A1- 3 233 419
- DE-A1- 3 330 813
- US-A- 4 057 055

## Beschreibung

Die Erfindung betrifft eine Vorrichtung zur Nagelkorrektur gemäß Oberbegriff des Hauptanspruchs.

Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese sogenannten Orthonyxiespangen reduzieren die Krümmung der Nagelplatte und entlasten dadurch den oftmals entzündeten Nagelfalz. Die Schmerzen werden deutlich gemindert und eine Abheilung wird ermöglicht. Zum Erzielen eines nachhaltigen Therapieerfolges verbleiben die Orthonyxiespangen für einen Zeitraum von mehreren Wochen bis einigen Monaten auf dem Nagel und unterstützen diesen in seinem geraden Wachstum.

Die Wirkung dieser Nagelkorrekturspangen beruht auf Rückstellkräften, außermittigen Zugkräften oder auf Kombinationen dieser beiden Mechanismen.

Folgende Vorrichtungen zur Nagelkorrektur sind bisher bekannt und werden zur Behandlung eingesetzt. Sie weisen jedoch entweder bezüglich ihrer Wirksamkeit oder ihrer Anwendung einige Nachteile auf:

### Drahtspange aus einem Teil (Fraserspange)

Die so genannte Fraserspange ist eine einteilige Drahtspange, versehen mit Häkchen, welche unter den Nagelrand greifen und diesen mittels Rückstellkraft anheben. Diese Spange muss vom Therapeuten für jeden Patienten individuell angefertigt werden. Dies erfolgt unter erheblichem Zeitaufwand an einem, zuvor anhand eines Abdruckes herzustellenden Modell. Die Vorrichtung wird von vorne auf den Nagel aufgeschoben, was eine exakte Anpassung auf die Nagelbreite erfordert und zudem ein erhebliches Verletzungsrisiko im engen oder gar entzündeten Nagelfalz darstellt. Die Wirkungskraft kann bei dieser Vorrichtung nur äußerst ungenau und nur durch sehr erfahrene Anwender eingestellt werden.

**Drahtspange aus 3 Teilen** (2 Schenkel + 1 Schlaufe; DE 42 07 797 A1)

Eine Weiterentwicklung der Fraserspange stellt die dreiteilige Drahtspange dar, welche aus zwei mit Häkchen versehenen Schenkeln und einer in der Mitte anzubringenden Drahtschlaufe besteht. Der Therapeut versieht die teilweise vorgefertigten Spangenschenkel mit Häkchen (oder passt diese im Falle einer industriellen Vorfertigung derselben an die Nageldicke an) und formt die Spange entsprechend der Nagelkrümmung. Die Schenkel werden einzeln unter den Nagelrand eingehängt und mit der vorgefertigten Schlaufe verbunden, wodurch die Spangenbreite an die Nagelbreite angepasst und die Spangenkraft stufenlos in Absprache mit dem Patienten eingestellt werden kann.

Von Nachteil sind auch bei dieser Drahtspange jedoch der oftmals zu enge Nagelfalz, welcher das Einhängen der Häkchen erschwert oder gar unmöglich macht, sowie die Gefahr von Verletzungen, vor allem beim Vorliegen von Entzündungen. Zudem erfordert die Anwendung großes Geschick des Therapeuten und macht eine spezielle Schulung unumgänglich.

**Klebespangen aus Kunststoff oder Metall** (DE 37 08 811 A1; BS-Spange)

Auf die Rückstellkraft von Metall- oder Kunststoffelementen basierende Klebespangen werden im vorgespannten Zustand auf die Nagelplatte aufgeklebt. Dafür muss die Spange an den Nagel angedrückt werden, was bei ohnehin schmerzhaft eingewachsenen Nägeln mit erheblichen Schmerzen für den Patienten verbunden sein kann. Bei zu geringem Anpressdruck hingegen ist die Spange nicht ausreichend fixiert und kann sich wieder lösen.

Je nach System wird die Intensität der Spangenkraft durch die Vorauswahl verschiedener Querschnitte oder durch Verringerung der Spangendicke im aufgeklebten Zustand variiert. Dies kann jedoch weder definiert noch stufenlos geschehen und ist außerdem nicht reversibel.

### Nagelkorrekturspange bestehend aus 2 Ankerplättchen mit einem losen Zugelement

Eine Vorrichtung, bestehend aus zwei auf den Nagel aufgeklebten Ankern, welche durch ein loses Zugelement miteinander verbunden werden, ist aus der DE 32 33 419 A1 bekannt. Hier können die Elemente zur Krafteinleitung im ungespannten Zustand auf die Nagelplatte geklebt werden. Die Einstellung der Spangenkraft erfolgt über ein elastisches Zugelement, wie zum Beispiel einen Gummiring, wobei die Intensität der Spangenkraft vom Abstand der

Befestigungselemente und der Größe und Stärke des Zugelements abhängt und daher nicht genau und stufenlos einstellbar ist.

Auf Grund der relativ hohen Ausführung der Ankerplatten kann durch enge Schuhe Druck auf den Nagel übertragen werden, zudem kann das nur lose befestigte Zugelement verloren werden.

Aufgabe der vorliegenden Erfindung ist es, die Nachteile des Stands der Technik zu überwinden und eine Nagelkorrekturspange zu schaffen, die ohne Schwierigkeiten, auch bei schmerzhaften Entzündungen, an die verschiedensten Krümmungen und Größen spannungsfrei auf dem Nagel angebracht werden kann und deren Wirkung erst im montierten Zustand aktiviert wird.

Im Laufe von Entwicklungsarbeiten im Rahmen der vorliegenden Erfindung wurde festgestellt, dass es von besonderem Vorteil ist, eine Nagelkorrekturvorrichtung so zu gestalten, dass durch Einwirkung einer stufenlos verstellbaren, definierbaren Kraft ein Biegemoment auf die Nagelplatte übertragen wird.

Der Gegenstand der Erfindung ist im Anspruch 1 definiert.

Fortentwicklungen und besondere Ausführungsformen der Erfindung ergeben sich aus den Unteransprüchen.

Nachstehend wird die Erfindung beispielhaft anhand der Zeichnungen näher erläutert:
Dabei zeigen:
   Fig. 1 eine erfindungsgemäße Vorrichtung zur Nagelkorrektur im montierten Zustand;
   Fig. 2 und 3 die erfindungsgemäße Vorrichtung zur Nagelkorrektur im Lieferzustand;
   Fig. 4 das Zugelement der erfindungsgemäßen Vorrichtung;
   Fig. 5 bis 8 das Anbringen und Aktivieren der Vorrichtung auf dem Nagel;
   Fig. 9 und 10 alternative Ausführungsformen der erfindungsgemäßen Vorrichtung.

Nachstehend wird auf die in den Figuren dargestellten Ausführungsformen näher eingegangen.

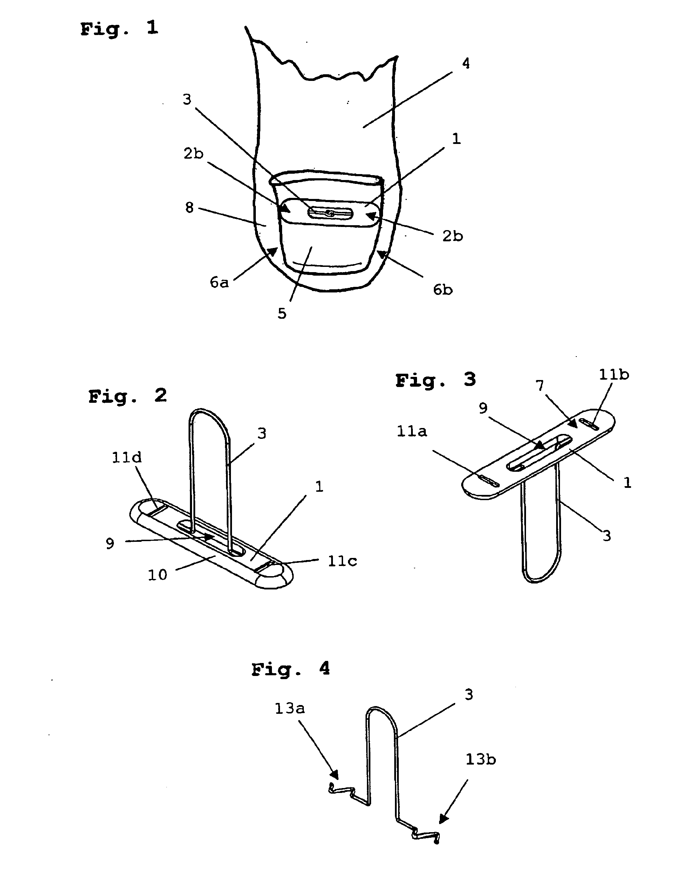

Figur 1 zeigt eine erfindungsgemäße Vorrichtung zur Nagelkorrektur im auf den Nagel montierten Zustand.

Zur Behandlung des eingewachsenen Nagels 5 einer Zehe 4 sollen die Nagelränder 6a, 6b minimal angehoben werden, um den Nagelfalz 8 zu entlasten. Hierfür wird ein Haftelement 1 mit einem schnell aushärtenden Klebstoff wie beispielsweise Cyanacrylat-Klebstoff auf den Nagel 5 aufgeklebt und dessen Randbereiche 2a, 2b mit einem Zugelement 3 aus Draht miteinander verbunden. Durch das definierte und stufenlose Verkürzen des Zugelements 3 und die Fixierung der eingestellten Position wird eine Zugkraft erzeugt, welche außermittig am Nagel 5 angreift. Auf den Nagel 5 wirkt dadurch ein Biegemoment und die Nagelränder 6a, 6b werden angehoben.

Die Figuren 2 und 3 zeigen die erfindungsgemäße Vorrichtung zur Nagelkorrektur in ihrem Lieferzustand. Das Zugelement 3 ist in das Haftelement 1 fest eingebettet und ragt lediglich im mittleren, im Haftelement 1 ausgesparten Bereich 9 als Bügel aus dem Haftelement 1 heraus.

Das Zugelement 3 besteht in der hier dargestellten Ausführung aus korrosionsbeständigem Stahldraht, welcher ein hohes Maß an plastischer Verformbarkeit aufweist. Alternativ ist auch eine Verwendung von Kunststofffaden als Material für das Zugelement möglich.

Das Haftelement 1 besteht aus Kunststoff, mit welchem das Zugelement 3 umspritzt wird. Durch die Verwendung eines gummielastischen Kunststoffes wirkt das Haftelement 1 als Federelement, was sich günstig auf die therapeutische Wirkung der Vorrichtung zur Nagelkorrektur auswirkt.

Anstatt aus Kunststoff, kann das Haftelement 1 auch aus Kautschuk oder Gummi bestehen und ebenso durch alternative Verfahren wie beispielsweise Kleben mit dem Zugelement 3 verbunden werden.

Zur Schonung des den Nagel umgebenden Fleisches, sowie zum Schutz vor unbeabsichtigtem Hängenbleiben und dadurch verursachtem Ablösen der Nagelkorrekturspange, sind die Oberkanten 10 des Haftelementes abgerundet.

Die Aussparung 9 im mittleren Bereich des Haftelementes 1 bietet nach der Montage der Vorrichtung auf dem Nagel Platz für die Umschlingung des verdrillten Zugelementes 3.

Die auf der Klebefläche 7 des Haftelementes 1 vorhandenen Vertiefungen 11a, 11b bilden Hinterschneidungen für eine verbesserte Querkraftaufnahme der Verklebung und dienen als Aufnahmekammer für den Klebstoff. Zusammen mit den Vertiefungen 11c, 11d auf der Oberseite des Haftelementes 1 dienen sie gleichzeitig zur Zentrierung des Zugelementes 3 im Haftelement 1 während des Fertigungsvorganges.

Zur Verwendung der erfindungsgemäßen Vorrichtung zur Nagelkorrektur für verschiedene Nagelbreiten ist diese in verschiedenen Längen ausgeführt, wobei der Drahtbügel des Zugelementes 3 im Hinblick auf ein einheitliches Spannwerkzeug bei allen Größen identisch ausgeführt werden sollte.

In Figur 4 ist das aus Draht gefertigte Zugelement 3 dargestellt. Um eine hohe Auszugsfestigkeit des Zugelementes 3 aus dem Haftelement zu gewährleisten, sind die vom Kunststoff umschlossenen Bereiche 13a, 13b des Zugelementes 3 gezackt ausgeführt. Alternativ zu einer zackenförmigen Ausführung der Enden des Zugelementes können auch andere Ausformungen wie Ösen oder Haken zu einer erhöhten Auszugsfestigkeit beitragen.

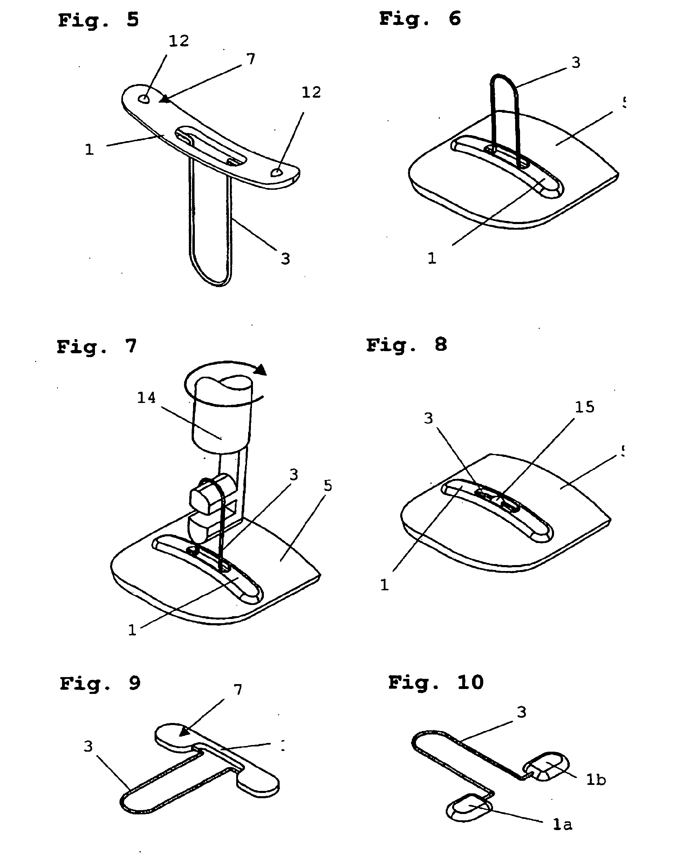

In den Figuren 5 bis 8 ist das Anbringen und Aktivieren der erfindungsgemäßen Nagelkorrekturspange dargestellt.

Wie in Figur 5 dargestellt, wird das Haftelement 1 der Nagelkorrekturspange entsprechend der Nagelwölbung gekrümmt und die Klebefläche 7 in ihren Randbereichen mit einem schnell aushärtenden Klebstoff 12 versehen. Anschließend wird die Vorrichtung zur Nagelkorrektur auf die Nagelplatte 5 aufgesetzt und mit dieser verklebt. Der Drahtbügel des Zugelementes 3 dient hierbei als Manipulationshilfe.

Die fertig mit der Nagelplatte 5 verklebte Nagelkorrekturvorrichtung ist in Figur 6 dargestellt.

Figur 7 zeigt das Verdrillen des Zugelementes 3 zum Aktivieren der auf dem Nagel 5 angebrachten Vorrichtung.

Zu diesem Zweck wird ein Werkzeug 14 in den Drahtbügel des Zugelementes 3 eingeführt und bis zum Erreichen der gewünschten Zugkraft verdrillt.

Die eingestellte Zugkraft bleibt durch die plastische Verformung des Drahtes im Bereich der Umschlingung des Zugelementes 3 erhalten. Bei der Verwendung alternativer Materialien oder der Aktivierung durch andere Spannverfahren muss die eingestellte Zugkraft gegebenenfalls durch form-, kraft- oder stoffschlüssige Verbindung des Zugelementes 3 zusätzlich fixiert werden.

Nach der Aktivierung kann die Drahtschlinge des Zugelementes 3 bis auf eine Umschlingung der entstandenen Spirale gekürzt werden. Die verbleibenden, spitzen Drahtenden werden wie in Figur 8 dargestellt mit einer beliebigen, zunächst modellierbaren und später aushärtenden Masse 15 abgedeckt und endgültig fixiert.

Die Figuren 9 und 10 zeigen alternative Ausführungsformen der erfindungsgemäßen Vorrichtung zur Nagelkorrektur.

Bei der in Figur 9 gezeigten Ausführung ragt das Zugelement 3 abweichend von der oben dargestellten Ausführung parallel zur Klebefläche 7 aus dem Haftelement 1 heraus. Das Haftelement 1 ist entsprechend auf der Seite des Austrittes des Zugelementes 3 ausgespart. Der Vorteil dieser Variante liegt in der noch flacheren Bauform der fertig angebrachten Vorrichtung zur Nagelkorrektur, da die beim Verdrillen des Zugelementes 3 entstehende Spirale nicht senkrecht sondern parallel zum Nagel liegt.

Figur 10 zeigt eine Ausführungsvariante der Vorrichtung zur Nagelkorrektur, bei welcher das Haftelement 1a, 1b zweiteilig ausgeführt ist. Dem Vorteil der möglichen Beschränkung auf eine Größe steht eine geringfügig erschwerte Handhabung beim Aufkleben entgegen. Die Zweiteilung des Haftelementes kann auch bei den oben dargestellten Ausführungen durch nachträgliches Zerschneiden während der Anwendung erfolgen.

Auch das Zugelement kann zweiteilig ausgeführt sein, wobei die einzeln aus dem Haftelement herausragenden Teile des Zugelementes an ihren Enden zur Aufnahme von Werkzeugen mit Ösen, Haken oder Ähnlichem versehen sein können.

### Vorteile der erfindungsgemäßen Vorrichtung zur Nagelkorrektur

Gegenüber den bisher bekannten, herkömmlichen Verfahren und Vorrichtungen weist die erfindungsgemäße Vorrichtung zur Nagelkorrektur einige Vorteile auf.

Durch ihre schnelle und einfache Anwendung wird diese Nagelkorrekturvorrichtung der Anforderung der Wirtschaftlichkeit in der ärztlichen, podologischen und kosmetischen Praxis besonders gerecht.

Durch die Vermeidung von Drahthäkchen, welche beim Einhängen in den oftmals entzündeten Nagelfalz stets mit einem Verletzungsrisiko behaftet sind, ist die Anwendung dieser Vorrichtung wesentlich sicherer als die herkömmlicher Drahtspangen. Diese Vorrichtung zur Nagelkorrektur kann daher auch anhand einer mitgelieferten Gebrauchsanweisung angewendet werden und erfordert deshalb keine spezifischen Vorkenntnisse.

Durch das Anbringen der Haftelemente im unbelasteten Zustand muss zum Aufkleben kein Schmerzen verursachender Druck auf den Nagel ausgeübt werden.

Die Zugkraft der Nagelkorrekturspange, und damit das Biegemoment auf den Nagel, kann stufenlos in Absprache mit dem Patienten eingestellt werden und ist bis zum Kürzen der Drahtspirale (siehe Fig. 8) reversibel.

Da die Aussparung im Haftelement der Nagelkorrekturvorrichtung die Drahtschlinge des Zugelementes aufnimmt, bleibt die Dicke der Vorrichtung auf die Höhe des Haftelementes beschränkt. Auf Grund dieser sehr flachen Bauweise wird sie auch in engen Schuhen nicht als störend empfunden und kann zudem durch eine geeignete Versiegelung optisch ansprechend gestaltet werden.

Die Abrundung der Oberkanten des Haftelementes ermöglicht dem Therapeuten, die Nagelkorrekturvorrichtung direkt am Rand des Nagels anzubringen, ohne eine Druckbelastung auf den Nagelfalz auszuüben. Die therapeutische Wirkung der Vorrichtung wird durch diesen Angriffspunkt möglichst weit außen am Nagel optimiert.

Der elastische Kunststoff des Haftelementes wirkt in Verbindung mit den in Zackenform ausgebildeten Enden des Zugelementes als Federelement im Kraftfluss. Dadurch bleibt die Nagelplatte trotz der fest angebrachten Vorrichtung zur Nagelkorrektur elastisch und kann sich beispielsweise beim Gehen in einem geringen Maß verformen. Dies wirkt sich sowohl hinsichtlich des Tragekomforts als auch auf die therapeutische Wirkung positiv aus.

## Patentansprüche

1. Vorrichtung zur Nagelkorrektur, bestehend aus einem Haftelement (1), welches durch Klebstoff auf einem Teilbereich der Nagelplatte (5) fixierbar ist, **dadurch gekennzeichnet, dass** mit einem aus dem Haftelement (1) austretenden Zugelement (3), welches in den Randbereichen (2a, 2b) des Haftelements (1) fest in das Material des Haftelementes (1) eingebettet ist, eine verstellbare, definierbare Zugkraft ausgeübt werden kann, welche durch außermittigen Kraftangriff ein Biegemoment auf die Nagelplatte überträgt.

2. Vorrichtung nach Anspruch 1, **dadurch gekennzeichnet, dass** das Haftelement einteilig ausgeführt ist.

3. Vorrichtung nach Anspruch 1, **dadurch gekennzeichnet, dass** das Haftelement mehrteilig ausgeführt ist.

4. Vorrichtung nach einem der vorstehenden Ansprüche, **dadurch gekennzeichnet, dass** das Haftelement eine Aussparung zur Aufnahme der Umschlingung des Zugelementes aufweist.

5. Vorrichtung nach einem der vorstehenden Ansprüche, **dadurch gekennzeichnet, dass** die Oberkanten des Haftelementes zum Schutz des den Nagel umgebenden Fleisches abgerundet sind.

6. Vorrichtung nach einem der vorstehenden Ansprüche, **dadurch gekennzeichnet, dass** die Vorrichtung zur Nagelkorrektur in mehreren unterschiedlichen Größen ausgeführt ist.

7. Vorrichtung nach einem der vorstehenden Ansprüche, **dadurch gekennzeichnet, dass** das Haftelement in der Klebefläche Vertiefungen zur Aufnahme des Klebstoffes sowie als Hinterschneidung aufweist.

8. Vorrichtung nach einem der vorstehenden Ansprüche, **dadurch gekennzeichnet, dass** die Zugkraft durch Verdrillen des Zugelementes ausgeübt werden kann.

9. Vorrichtung nach einem der Ansprüche 1 bis 7, **dadurch gekennzeichnet, dass** die Zugkraft mit Hilfe eines entsprechenden Werkzeugs ausgeübt werden kann.

10. Vorrichtung nach einem der vorstehenden Ansprüche, **dadurch gekennzeichnet, dass** die nach Ausübung der Zugkraft erreichte Position durch Formschluss fixierbar ist.

11. Vorrichtung nach einem der vorstehenden Ansprüche, **dadurch gekennzeichnet, dass** das Zugelement aus Draht besteht.

12. Vorrichtung nach einem der vorstehenden Ansprüche, **dadurch gekennzeichnet, dass** das Haftelement aus einem elastischen Kunststoff besteht.

## Claims

1. Device for nail correction comprising a bonding member (1) which is adapted to be fixed on a portion of the nail body (5) by adhesive, **characterized in that** with a tension member (3) which extends from the bonding member (1) and is fixedly embedded in the material of said bonding member (1) in the edge regions (2a, 2b) of said bonding member (1) an adjustable definable tensile force can be exerted which by eccentric force application transmits a bending moment to the nail body.

2. Device according to claim 1, **characterized in that** the bonding member is formed as one-part construction.

3. Device according to claim 1, **characterized in that** the bonding member is formed as multipart construction.

4. Device according to any one of the preceding claims, **characterized in that** the bonding member comprises a recess for receiving the loop of the tension member.

5. Device according to any one of the preceding claims, **characterized in that** the upper edges of the bonding member are rounded to protect the flesh surrounding the nail.

6. Device according to any one of the preceding claims, **characterized in that** the device for nail correction is made in several different sizes.

7. Device according to any one of the preceding claims, **characterized in that** the bonding member comprises in the bonding surface depressions for receiving the adhesive and as undercutting.

8. Device according to any one of the preceding claims, **characterized in that** the tensile force can be exerted by twisting the tension member.

9. Device according to any one of claims 1 to 7, **characterized in that** the tensile force can be exerted with the aid of a corresponding tool.

10. Device according to any one of the preceding claims, **characterized in that** the position reached after exertion of the tensile force can be fixed by form-locking.

11. Device according to any one of the preceding claims, **characterized in that** the tension member consists of wire.

12. Device according to any one of the preceding claims, **characterized in that** the bonding member consists of a resilient plastic.

## Revendications

1. Dispositif destiné à la correction d'ongles, se composant d'un élément adhésif (1), lequel peut être fixé au moyen d'une colle sur une zone partielle de la plaque ungéale (5), **caractérisé en ce qu'**une force de traction définie et ajustable peut être exercée avec un élément de traction (3) sortant de l'élément adhésif (1), lequel élément de traction est intégré fixement dans le matériau de l'élément adhésif (1) au niveau des zones périphériques (2a, 2b) de l'élément adhésif (1), laquelle force de traction transmet à la plaque ungéale un moment de flexion grâce à une application de force excentrique.

2. Dispositif selon la revendication 1, **caractérisé en ce que** l'élément adhésif est réalisé d'un seul tenant.

3. Dispositif selon la revendication 1, **caractérisé en ce que** l'élément adhésif est réalisé en plusieurs pièces.

4. Dispositif selon l'une quelconque des revendications précédentes, **caractérisé en ce que** l'élément adhésif présente un évidement servant à recevoir la boucle de l'élément de traction.

5. Dispositif selon l'une quelconque des revendications précédentes, **caractérisé en ce que** les arêtes supérieures de l'élément adhésif sont arrondies pour la protection de la chair entourant l'ongle.

6. Dispositif selon l'une quelconque des revendications précédentes, **caractérisé en ce que** le dispositif servant à corriger l'ongle est réalisé en différentes tailles.

7. Dispositif selon l'une quelconque des revendications précédentes, **caractérisé en ce que** l'élément adhésif présente au niveau de la surface adhésive des creux servant à recevoir de la colle et servant également de contre-dépouille.

8. Dispositif selon l'une quelconque des revendications précédentes, **caractérisé en ce que** la force de traction peut être exercée par torsion de l'élément de traction.

9. Dispositif selon l'une quelconque des revendications 1 à 7, **caractérisé en ce que** la force de traction peut être exercée à l'aide d'un outil approprié.

10. Dispositif selon l'une quelconque des revendications précédentes, **caractérisé en ce que** la position atteinte après application de la force de traction peut être fixée par complémentarité de forme.

11. Dispositif selon l'une quelconque des revendications précédentes, **caractérisé en ce que** l'élément de traction est en fil d'acier.

12. Dispositif selon l'une quelconque des revendications précédentes, **caractérisé en ce que** l'élément adhésif est en une matière plastique élastique.
